# EUROPÄISCHE PATENTANMELDUNG

(11) **EP 3 339 274 A1**
(43) Veröffentlichungstag der Anmeldung: **27.06.2018**
(21) Anmeldenummer: 16206418.2
(22) Anmeldetag: 22.12.2016
(51) Int. Cl.: C07C 5/48, C07C 11/04

(54) **VERFAHREN UND ANLAGE ZUR HERSTELLUNG VON OLEFINEN**

(71) Anmelder: Linde Aktiengesellschaft, 80331 München (DE)
(72) Erfinder: Zellhuber, Mathieu, 82152 Martinsried (DE); Peschel, Andreas, 82515 Wolfratshausen (DE); Fritz, Helmut, 81375 München (DE)
(74) Vertreter: m patent group

(57) **Zusammenfassung**

Es wird ein Verfahren zur Herstellung von Ethylen vorgeschlagen, bei dem ein Ethan und Sauerstoff enthaltender Reaktionseinsatz gebildet und ein Teil des Ethans und des Sauerstoffs in dem Reaktionseinsatz unter Erhalt eines Prozessgases durch oxidative Dehydrierung zu Ethylen und zu Essigsäure umgesetzt wird, wobei das Prozessgas den nicht umgesetzten Teil des Ethans und des Sauerstoffs, das Ethylen und die Essigsäure sowie Wasser enthält, und wobei das Prozessgas einer Wasserwäsche unterworfen wird. Es ist vorgesehen, dass die Wasserwäsche umfasst, das Prozessgas in eine Waschkolonne (10, 20, 30, 40, 50) einzuspeisen, in die in wenigstens zwei unterschiedlichen Kolonnenteilen jeweils wässrige, flüssige Waschmittelströme eingespeist und dem Prozessgas entgegengeschickt werden. Eine entsprechende Anlage (100) ist ebenfalls Gegenstand der vorliegenden Erfindung.

## Beschreibung

Die Erfindung betrifft ein Verfahren zur Herstellung von Olefinen und eine entsprechende Anlage gemäß den Oberbegriffen der unabhängigen Patentansprüche.

### Stand der Technik

Die oxidative Dehydrierung (engl. Oxidative Dehydrogenation, ODH) von Paraffinen mit zwei bis vier Kohlenstoffatomen ist grundsätzlich bekannt. Bei der ODH werden die genannten Paraffine mit Sauerstoff unter anderem zu den Olefinen gleicher Kohlenstoffzahl und zu Wasser umgesetzt.

Die ODH kann gegenüber etablierteren Verfahren zur Herstellung von Olefinen wie dem Steamcracken oder der katalytischen Dehydrierung vorteilhaft sein. So besteht aufgrund der Exothermie der beteiligten Reaktionen keine thermodynamische Gleichgewichtslimitierung. Die Bildungsenergien ΔG für Ethan, Propan bzw. n-Butan betragen -102, -115 bzw. -118 kJ/mol. Die ODH kann bei vergleichsweise geringen Reaktionstemperaturen durchgeführt werden. Grundsätzlich ist keine Regenerierung der eingesetzten Katalysatoren erforderlich, da die Anwesenheit von Sauerstoff eine insitu-Regenerierung ermöglicht. Schließlich werden im Gegensatz zum Steamcracken geringere Mengen an wertlosen Nebenprodukten wie Koks gebildet.

Zu weiteren Details bezüglich der ODH sei auf einschlägige Fachliteratur, beispielsweise Ivars, F. und López Nieto, J. M., Light Alkanes Oxidation: Targets Reached and Current Challenges, in: Duprez, D. und Cavani, F. (Hrsg.), Handbook of Advanced Methods and Processes in Oxidation Catalysis: From Laboratory to Industry, London 2014: Imperial College Press, Seiten 767-834, oder Gärtner, C.A. et al., Oxidative Dehydrogenation of Ethane: Common Principles and Mechanistic Aspects, ChemCatChem, Bd. 5, Nr. 11, 2013, Seiten 3196 bis 3217, verwiesen.

Die Erfindung wird nachfolgend insbesondere unter Bezugnahme auf die ODH von Ethan (sogenannte ODH-E) beschrieben. Ihr Einsatz ist jedoch grundsätzlich auch bei der ODH höherer Paraffine wie Propan und Butan möglich und vorteilhaft.

Bei der ODH werden insbesondere bei Verwendung von MoVNbTeOx-Katalysatoren unter industriell relevanten Reaktionsbedingungen signifikante Mengen der jeweiligen Carbonsäuren der eingesetzten Paraffine als Nebenprodukte gebildet. Für einen wirtschaftlichen Anlagenbetrieb ist eine entsprechende Koppelproduktion von Olefinen und der jeweiligen Carbonsäuren bei Verwendung des beschriebenen Katalysatortyps in der Regel erforderlich. Dies gilt insbesondere für die ODH-E, bei der gleichzeitig Essigsäure gebildet wird. Zudem werden bei der ODH nennenswerte Mengen an u.a. Kohlenmonoxid und Kohlendioxid als Nebenprodukte gebildet, die zusammen mit Wasser, Carbonsäuren, Restsauerstoff und Restethan in einem in der ODH gebildeten Gasgemisch enthalten sind und in von den jeweiligen Hauptprodukten, also dem oder den erzeugten Olefinen, abgetrennt werden müssen.

Aus der US 2014/0249339 A1 ist bekannt, ein entsprechendes Gasgemisch einer Wasserwäsche zu unterwerfen, um dieses zu kühlen und wasserlösliche Komponenten aus diesem auszuwaschen. Die vorliegende Erfindung stellt sich die Aufgabe, entsprechende Wasserwäschen zu verbessern.

### Offenbarung der Erfindung

Vor diesem Hintergrund schlägt die vorliegende Erfindung ein Verfahren zur Herstellung von Olefinen und eine entsprechende Anlage mit den Merkmalen der unabhängigen Patentansprüche vor. Ausgestaltungen sind jeweils Gegenstand der abhängigen Patentansprüche sowie der nachfolgenden Beschreibung.

Stoffströme, Gasgemische usw. können im hier verwendeten Sprachgebrauch reich oder arm an einer oder mehreren Komponenten sein, wobei die Angabe "reich" für einen Gehalt von wenigstens 99%, 99,5%, 99,9% oder 99,99% und die Angabe "arm" für einen Gehalt von höchstens 1%, 0,5%, 0,1% oder 0,01% auf molarer, Gewichts- oder Volumenbasis stehen kann. Sind mehrere Komponenten angegeben, bezieht sich die Angabe "reich" oder "arm" auf die Summe aller Komponenten. Ist hier beispielsweise von "Sauerstoff" oder "Ethan" die Rede, kann es sich um ein Reingas, aber auch um ein an der jeweiligen Komponente reiches Gemisch handeln. Stoffströme, Gasgemische usw. können im hier verwendeten Sprachgebrauch außerdem "angereichert" oder "abgereichert" an einer oder mehreren Komponenten sein, wobei sich diese Begriffe auf einen Gehalt in einem Ausgangsgemisch beziehen. Sie sind "angereichert", wenn sie zumindest den 1,5-fachen, 2-fachen, 5-fachen, 10-fachen, 100-fachen oder 1.000-fachen Gehalt, "abgereichert", wenn sie höchstens den 0,75-fachen, 0,5-fachen, 0,1-fachen, 0,01-fachen oder 0,001-fachen Gehalt einer oder mehrerer Komponenten, bezogen auf das Ausgangsgemisch, enthalten.

Nachfolgend werden zur Charakterisierung von Drücken und Temperaturen die Begriffe "Druckniveau" und "Temperaturniveau" verwendet, wodurch zum Ausdruck gebracht werden soll, dass Drücke und Temperaturen nicht in Form exakter Druck- bzw. Temperaturwerte vorliegen werden müssen. Ein Druckniveau oder Temperaturniveau kann beispielsweise ± 1%, 5%, 10%, 20% oder 50% um einen Mittelwert liegen. Mehrere Druck- und Temperaturniveaus können disjunkte oder überlappende Bereiche darstellen. Dasselbe Druck- bzw. Temperaturniveau kann beispielsweise auch dann noch vorliegen, wenn sich aufgrund von Leitungsverlusten oder Abkühlung Drücke und Temperaturen reduziert haben. Bei hier in bar angegebenen Druckniveaus handelt es sich um Absolutdrücke.

Bei einer "Rektifikationskolonne" handelt es sich im hier verwendeten Sprachgebrauch um eine Trenneinheit, die dafür eingerichtet ist, ein gasförmig oder flüssig oder in Form eines Zweiphasengemischs mit flüssigen und gasförmigen Anteilen, ggf. auch im überkritischen Zustand, eingespeistes Stoffgemisch durch Rektifikation zumindest teilweise aufzutrennen, also aus dem Stoffgemisch jeweils Reinstoffe oder zumindest Stoffgemische mit anderer Zusammensetzung zu erzeugen. Typischerweise sind Rektifikationskolonnen als zylindrische Metallbehälter ausgebildet, die mit Einbauten, beispielsweise Trennböden oder geordneten oder ungeordneten Packungen, ausgerüstet sind. Eine Rektifikationskolonne weist einen Sumpfverdampfer auf. Hierbei handelt es sich um eine Einrichtung mit einem Wärmetauscher, der beheizt wird und dafür eingerichtet ist, eine im Sumpf der Rektifikationskolonne anfallende flüssige Fraktion, auch als Sumpfflüssigkeit bezeichnet, zu erwärmen. Mittels eines Sumpfverdampfers wird kontinuierlich ein Teil des Sumpfprodukts verdampft und gasförmig in dem Trennbereich zurückgespeist.

Unter "inerten Komponenten" seien hier in der ODH nichtreaktive, nichtkondensierte gasförmige Anteile eines Gasgemischs verstanden, beispielsweise Edelgase wie Argon, aber auch Verbindungen wie Methan.

Wie eingangs erwähnt, können in der ODH insbesondere bei Verwendung von MoVNbTeOx-Katalysatoren signifikante Mengen der jeweiligen Carbonsäuren der eingesetzten Paraffine als Nebenprodukte gebildet werden. Weitere Nebenprodukte sind unter anderem Kohlenmonoxid und Kohlendioxid. Ferner enthält ein einem ODH-Reaktor entnommenes Gasgemisch typischerweise Reaktions- und Prozesswasser, Restsauerstoff und Restethan. Ein solches Gasgemisch wird hier als "Prozessgas" der ODH bezeichnet. Das Prozessgas enthält, wie erwähnt, ein oder mehrere Olefine als Hauptprodukt(e) sowie Nebenprodukte und nicht umgesetzte Edukte.

### Vorteile der Erfindung

Wie eingangs erwähnt, sind zur Bearbeitung von Prozessgasen aus der ODH Wasserwäschen bekannt. Insbesondere dann, wenn entsprechende Gasgemische nennenswerte Mengen an einer oder mehreren Carbonsäuren wie Essigsäure enthalten, erweisen sich bekannte Verfahren aber als nicht befriedigend, weil die Carbonsäure(n) nur unzureichend ausgewaschen wird bzw. werden. Dies kann insbesondere auf nachgeschaltete Verfahrensschritte negative Auswirkungen haben. Diese werden nachfolgend unter Bezugnahme auf Essigsäure, und damit das Beispiel der ODH-E, beschrieben. Entsprechende Erläuterungen gelten jedoch (mit Einschränkungen) auch für höhere Carbonsäuren.

Essigsäure und Wasser weisen sehr ähnliche Siedepunkte auf und sind dementsprechend schwer destillativ voneinander zu trennen. Dies führt dazu, dass bei unzureichender Abtrennung auch bei der weiteren Bearbeitung des Prozessgases eine Kondensation von Wasser und Essigsäure eintreten kann, die weitgehend simultan abläuft. Ein wässriges Kondensat wird daher stets einen nennenswerten Säureanteil aufweisen, der in der Nähe des Essigsäure-Wasser- Verhältnisses im Prozessgas direkt stromab des oder der verwendeten Reaktoren liegen wird.

Mit anderen Worten können bei einer unzureichenden Abtrennung der Essigsäure säurehaltige Kondensate in weiten Teilen der Trennung bzw. in darin verwendeten Einrichtungen auftreten. Bei Anwendung eines für Olefinanlagen typischen Trennkonzepts hieße das, dass in der kompletten Quench- und Verdichtersektion teilweise hohe Essigsäurekonzentrationen auftreten können. Dies erfordert einen Mehraufwand bei Materialauswahl und/oder kann die Langlebigkeit entsprechender Anlagen deutlich reduzieren.

Hinzu kommt, dass möglicherweise vorgesehene Prozesseinheiten zur Kohlendioxidabtrennung, beispielsweise eine Amin- und/oder einer Laugenwäsche, in ihrer Funktionsweise von nennenswerten Essigsäuremengen im Produktgas negativ beeinflusst werden.

Durch das erfindungsgemäß vorgesehene Verfahren wird erreicht, dass bereits im ersten bzw. einem frühen Schritt einer entsprechenden Trennung eine weitgehende Entfernung der bei der ODH gebildeten Essigsäure erfolgen kann. Das erfindungsgemäße Verfahren stellt ferner eine gute Wärmeintegration sowie einen geringen Druckverlust im System sicher.

Zur Erzielung dieser Vorteile geht die vorliegende Erfindung von einem grundsätzlich bekannten Verfahren zur Herstellung von Ethylen aus, bei dem ein Ethan und Sauerstoff enthaltender Reaktionseinsatz gebildet und ein Teil des Ethans und des Sauerstoffs in dem Reaktionseinsatz unter Erhalt eines Prozessgases durch oxidative Dehydrierung zu dem Ethylen und zu Essigsäure umgesetzt wird, wobei das Prozessgas den nicht umgesetzten Teil des Ethans und des Sauerstoffs, das Ethylen und die Essigsäure sowie Wasser enthält, und wobei das Prozessgas einer Wasserwäsche unterworfen wird. Details wurden bereits erläutert.

Erfindungsgemäß ist nun vorgesehen, dass die Wasserwäsche umfasst, das Prozessgas in eine Waschkolonne einzuspeisen, in die in wenigstens zwei unterschiedlichen Kolonnenteilen jeweils wässrige, flüssige Waschmittelströme eingespeist und dem Prozessgas entgegengeschickt werden. Auf diese Weise kann eine verbesserte Wärmeintegration erzielt werden, indem eine selektive Temperierung der unterschiedlichen Waschmittelströme vorgenommen wird, und der Gehalt an Essigsäure kann, insbesondere auch in den nachfolgend erläuterten Ausgestaltungen, auf für nachgeschaltete Einheiten akzeptable Werte verringert werden. In jedem Fall wird vom Kopf der Waschkolonne ein insbesondere gekühltes und von Essigsäure zumindest weitgehend gereinigtes Prozessgas abgezogen.

Gemäß einer bevorzugten Ausgestaltung des erfindungsgemäßen Verfahrens wird eine Waschkolonne mit einem ersten Kolonnenteil und einem zweiten Kolonnenteil verwendet, wobei der zweite Kolonnenteilen oberhalb des ersten Kolonnenteils angeordnet ist, das Prozessgas in einen unteren Bereich des ersten Kolonnenteils eingespeist und teilweise oder vollständig durch den ersten Kolonnenteil, von dem ersten Kolonnenteil in den zweiten Kolonnenteil und durch den zweiten Kolonnenteil aufsteigen gelassen wird, und in einem oberen Bereich des ersten Kolonnenteils ein erster flüssiger Waschmittelstrom und in einem oberen Bereich des zweiten Kolonnenteils ein zweiter flüssiger Waschmittelstrom eingespeist wird.

Unter "unterschiedlichen Kolonnenteilen" werden im hier verwendeten Sprachgebrauch Bereiche einer Waschkolonne, die keine räumliche Überschneidung miteinander aufweisen, verstanden. Insbesondere sind unterschiedliche Kolonnenteile in unterschiedlicher Höhe angeordnete Bereiche. Die unterschiedlichen Kolonnenteile weisen dabei (gleiche oder unterschiedliche) Austauschstrukturen auf, die insbesondere dafür eingerichtet sind, eine Oberfläche des in den jeweiligen Kolonnenteilen herabrieselnden Waschmittels zu vergrößern und damit einen verbesserten Austausch mit dem Prozessgas sicherzustellen. Insbesondere können die Kolonnenteile hierzu die bereits erwähnten Einbauten, beispielsweise Trennböden oder geordneten oder ungeordnete Packungen, aufweisen.

Unter einem "unteren" Bereich eines Kolonnenteils wird hier ein Bereich verstanden, der bei weniger als 50%, insbesondere weniger als 40%, 30%, 20% oder 10% der Höhe des jeweiligen Kolonnenteils endet. Entsprechend wird hier unter einem "oberen" Bereich eines Kolonnenteils ein Bereich verstanden, der bei mehr als 50%, 60%, 70%, 80% oder 90% der Höhe des Kolonnenteils beginnt.

Vorteilhafterweise werden der erste Waschmittelstrom auf einem ersten Temperaturniveau in den ersten Kolonnenteil und der zweite Waschmittelstrom auf einem zweiten Temperaturniveau in den zweiten Kolonnenteil eingespeist. Insbesondere kann dabei das erste Temperaturniveau bei 50 bis 90 °C und/oder das zweite Temperaturniveau bei 20 bis 50 °C liegen. Be i derartigen Temperaturen kann eine vorteilhafte Wärmeintegration erfolgen, indem zur Bereitstellung des ersten Temperaturniveaus beispielsweise ein in einer entsprechenden Anlage strömender Stoffstrom und zur Bereitstellung des zweiten Temperaturniveaus Kühlwasser zumindest teilweise verwendet werden. Grundsätzlich können im Rahmen der vorliegenden Erfindung wärmeintegrierte Wärmetauscher, d.h. mit in einer entsprechenden Anlage strömenden Stoffströmen wie Einsatz- oder Produktströmen betriebene Wärmetauscher, und sogenannte "Utility"-Wärmetauscher, d.h. Wärmetauscher, die mit einem separaten Medium betrieben werden, jeweils alleine und in beliebiger Kombination miteinander eingesetzt werden.

Im Rahmen der vorliegenden Erfindung kann vorgesehen sein, dass das Prozessgas in dem ersten (unteren) Kolonnenteil mit der gesamten (vereinigten) Flüssigkeit beider Waschmittelströme und in dem zweiten (oberen) Kolonnenteil nur mit der Flüssigkeit des zweiten Waschmittelstroms in Kontakt gebracht wird. Es ist jedoch alternativ zu dieser ersten Variante gemäß einer zweiten Variante auch möglich, das Prozessgas in dem ersten Kolonnenteil nur mit der Flüssigkeit des ersten Waschmittelstroms und in dem zweiten Kolonnenteil nur mit der Flüssigkeit des zweiten Waschmittelstroms in Kontakt zu bringen. In der zweiten Variante ist es dabei möglich, dass der erste und der zweite Waschmittelstrom unterschiedliche Gehalte an insbesondere Essigsäure aufweisen. Insbesondere kann der zweite Waschmittelstrom einen geringeren Gehalt an Essigsäure aufweisen als der erste, so dass eine verbesserte Auswaschung vorgenommen werden kann.

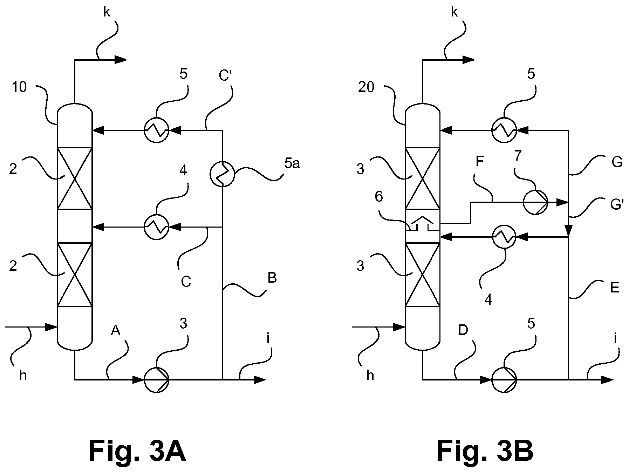

Die soeben erläuterte erste Variante umfasst insbesondere, dass aus einem unteren Bereich des ersten Kolonnenteils Flüssigkeit entnommen und teilweise oder vollständig zur Bildung des ersten und des zweiten Waschmittelstroms verwendet wird. Mit anderen Worten kann entsprechende Flüssigkeit also aufgeteilt und zum Teil in Form oder als Teil des ersten und zum Teil in Form oder als Teil des zweiten Waschmittelstroms eingesetzt werden. Aufgrund dieser Bildung des ersten und des zweiten Waschmittelstroms weisen diese insbesondere dieselben Gehalte an Essigsäure und von anderen Verbindungen auf. Diese Variante ist insbesondere unter Bezugnahme auf die beigefügte Figur 3A veranschaulicht.

Die soeben erläuterte zweite Variante wird insbesondere dadurch realisiert, dass der erste Kolonnenteil durch einen Flüssigkeitssperrboden von dem zweiten Kolonnenteil getrennt ist, und insbesondere aus einem unteren Bereich des ersten Kolonnenteils Flüssigkeit entnommen und teilweise oder vollständig bei der Bildung des ersten Waschmittelstroms verwendet wird, und aus einem unteren Bereich des zweiten Kolonnenteils Flüssigkeit entnommen wird.

Die aus dem unteren Bereich des zweiten Kolonnenteils entnommene Flüssigkeit kann in der zweiten Variante teilweise oder vollständig bei der Bildung des zweiten Waschmittelstroms verwendet werden. Wird die aus dem unteren Bereich des zweiten Kolonnenteils entnommene Flüssigkeit in der zweiten Variante nicht oder nicht vollständig bei der Bildung des zweiten Waschmittelstroms verwendet, kann die aus dem unteren Bereich des zweiten Kolonnenteils entnommene Flüssigkeit oder ein verbleibender Anteil auch ausgeführt oder bei der Bildung des ersten Waschmittelstroms verwendet werden. Der zweite Waschmittelstrom kann in der zweiten Variante insbesondere auch derart gebildet werden, dass er keine aus dem unteren Bereich des zweiten Kolonnenteils entnommene Flüssigkeit umfasst. Auf diese Weise kann ein Einmaldurchlauf des zweiten Waschmittelstroms erzielt werden.

In sämtlichen erläuterten Fällen kann der zweite Waschmittelstrom Frischwasser, also beispielsweise demineralisiertes Wasser, und/oder aufgereinigtes Wasser aus einer weiteren Prozesseinheit, insbesondere einer Essigsäurerückgewinnungseinheit, umfassen. Wird der zweite Waschmittelstrom, wie erläutert, derart gebildet, dass er keine aus dem unteren Bereich des zweiten Kolonnenteils entnommene Flüssigkeit umfasst, kann der zweite Waschmittelstrom auch vollständig aus solchem Frisch- bzw. demineralisiertem Wasser und/oder aufgereinigtem Wasser bestehen. Auch eine Zumischung weiterer Flüssigkeiten zu dem zweiten Waschmittelstrom ist möglich. Aufgereinigtes Wasser aus einer Essigsäurerückgewinnungseinheit enthält beispielsweise ca. 0 bis 5.000 Gew.-ppm Essigsäure.

Durch die Verwendung des Flüssigkeitssperrbodens, durch den Gas aufsteigen, aber keine Flüssigkeit nach unten ablaufen kann, wird in der zweiten Variante verhindert, dass sich Flüssigkeit des ersten und des zweiten Waschmittelstroms nennenswert und unkontrolliert in der Kolonne vermischen. Diese Variante ist insbesondere unter Bezugnahme auf die beigefügte Figur 3B veranschaulicht.

Die beiden Waschmittelströme können in dieser zweiten Variante daher die zuvor erläuterten unterschiedlichen Gehalte aufweisen. In aufsteigender Richtung kann auf diese Weise ein zunehmend reines Waschmittel eingesetzt werden. Mit anderen Worten können der erste und der zweite Waschmittelstrom überwiegend Wasser enthalten, und der erste Waschmittelstrom kann einen höheren Essigsäuregehalt aufweisen als der zweite Waschmittelstrom.

Eine Erweiterung der erläuterten ersten oder der erläuterten zweiten Variante, hier als dritte Variante bezeichnet, umfasst, eine Waschkolonne mit einem dritten Kolonnenteil oberhalb des zweiten Kolonnenteils zu verwenden, wobei das Prozessgas teilweise oder vollständig von dem zweiten Kolonnenteil in den dritten Kolonnenteil und durch den dritten Kolonnenteil aufsteigen gelassen wird, in einem oberen Bereich des dritten Kolonnenteils ein dritter flüssiger Waschmittelstrom eingespeist wird, und insbesondere aus einem unteren Bereich des dritten Kolonnenteils Flüssigkeit entnommen wird. In dieser dritten Variante kann ein nochmals reineres Kopfprodukt der Waschkolonne erhalten werden. Hierbei ist zwischen dem zweiten und dem dritten Kolonnenteil insbesondere ein oder ein weiterer Flüssigkeitssperrboden angeordnet.

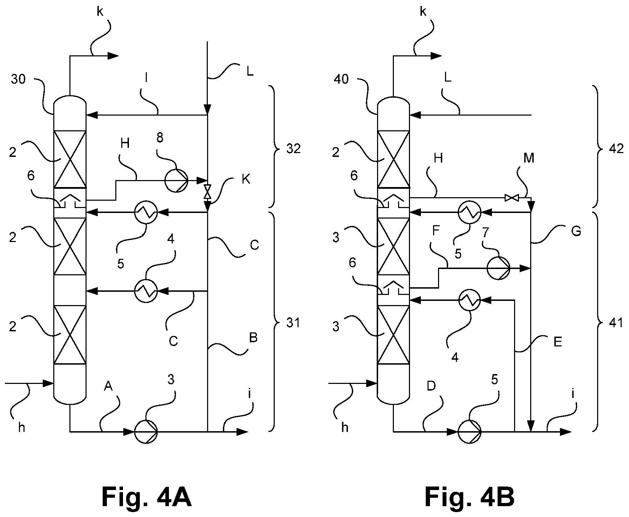

In der dritten Variante ist eine erste Ausgestaltung möglich, bei der ein Einmaldurchlauf des dritten Waschmittelstroms vorgenommen wird, also am Kopf der Waschkolonne der dritte Waschmittelstrom eingespeist und aus einem unteren Bereich des dritten Kolonnenteils, oberhalb des Flüssigkeitssperrbodens, Flüssigkeit abgezogen aber nicht mehr im Kreislauf geführt, d.h. bei der Bildung des dritten Waschmittelstroms, verwendet wird. Die Flüssigkeit kann beispielsweise bei der Bildung des zweiten Waschmittelstroms verwendet und/oder ausgeführt werden. Auf diese Weise kann insbesondere stets ein hochreiner dritter Waschmittelstrom bereitgestellt werden. Dies ist insbesondere unter Bezugnahme auf Figur 4B veranschaulicht. Eine zweite Ausgestaltung der dritte Variante sieht hingegen einen entsprechenden Kreislauf vor, wie insbesondere auch in Figur 4A gezeigt.

In der ersten Ausgestaltung der dritten Variante wird die aus dem unteren Bereich des dritten Kolonnenteils entnommene Flüssigkeit also insbesondere teilweise oder vollständig bei der Bildung des zweiten Waschmittelstroms verwendet oder ausgeführt. In der zweiten Ausgestaltung der dritten Variante wird hingegen die aus dem unteren Bereich des dritten Kolonnenteils entnommene Flüssigkeit teilweise oder vollständig bei der Bildung des dritten Waschmittelstroms verwendet. In jedem Fall enthält der dritte Waschmittelstrom überwiegend Wasser und vorteilhafterweise höchstens 5.000 Gew.-ppm Essigsäure.

In der ersten Ausgestaltung der dritten Variante kann der dritte Waschmittelstrom aus Frischwasser, also beispielsweise demineralisiertem Wasser, oder aber auch beispielsweise aus dem erwähnten aufgereinigtem Wasser aus einer weiteren Prozesseinheit, insbesondere einer Essigsäurerückgewinnungseinheit, gebildet sein oder der dritte Waschmittelstrom kann zumindest solches Wasser umfassen. Zu Details sei auf die entsprechenden Ausführungen zur zweiten Variante verwiesen. Aber auch in der zweiten Ausgestaltung der dritten Variante kann der dritte flüssige Waschmittelstrom neben der aus dem unteren Bereich des dritten Kolonnenteils entnommenen Flüssigkeit entsprechendes Wasser umfassen.

In der dritten Variante, insbesondere in der ersten Ausgestaltung, kann in dem dritten Kolonnnenteil auch beispielsweise eine chemische Wäsche unter Verwendung von Lauge durchgeführt werden. Es kann also ein wässriger Laugen- oder Ablaugenstrom, d.h. eine alkalische Lösung mit einer oder mehreren geeigneten organischen oder anorganischen Komponenten, als der dritte Waschmittelstrom oder als Teil des dritten Waschmittelstroms, verwendet werden. Hierbei kann entweder Frischlauge, beispielsweise wässrige Natronlauge, oder Ablauge aus einer nachgeschalteten Laugenwäsche zur Bearbeitung des Prozessgases und/oder eine Mischung der genannten Flüssigkeiten verwendet werden. Auf diese Weise kann insbesondere eine weitere Nutzung entsprechender Ablauge aus einer Laugenwäsche vorgenommen werden, bevor diese einer Ablaugeeinheit zugeführt wird. Hierdurch verbessert sich die Ausnutzung der Lauge.

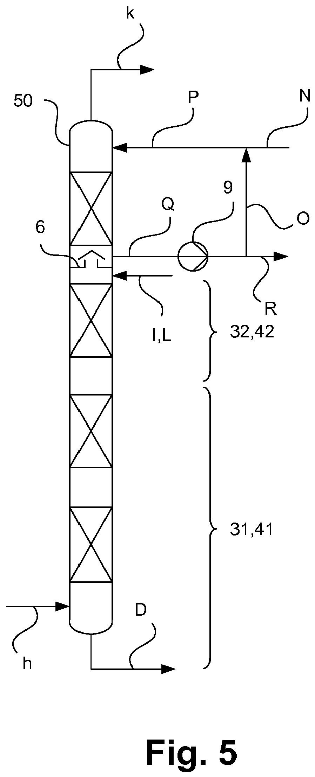

Eine Erweiterung der erläuterten dritten Variante, hier als vierte Variante bezeichnet, sieht vor, dass eine Waschkolonne mit einem vierten Kolonnenteil oberhalb des dritten Kolonnenteils verwendet wird, wobei das Prozessgas teilweise oder vollständig von dem dritten Kolonnenteil in den vierten Kolonnenteil und durch den vierten Kolonnenteil aufsteigen gelassen wird, in einem oberen Bereich des vierten Kolonnenteils ein vierter flüssiger Waschmittelstrom eingespeist wird, und aus einem unteren Bereich des vierten Kolonnenteils Flüssigkeit entnommen wird. In dieser Ausgestaltung kann in dem obersten (vierten) Kolonnnenteil insbesondere die chemische Wäsche unter Verwendung von Lauge durchgeführt werden, wie sie bereits unter Bezugnahme auf die zweite Variante erläutert wurde. Es wird also auch hier insbesondere ein wässriger Laugen- oder Ablaugenstrom, d.h. eine alkalische Lösung einer oder mehrerer geeigneter organischer oder anorganischer Komponenten, als der vierte Waschmittelstrom oder als Teil hiervon verwendet. Es kann auch hier entweder Frischlauge, beispielsweise wässrige Natronlauge, oder Ablauge aus einer nachgeschalteten Laugenwäsche zur Bearbeitung des Prozessgases und/oder eine Mischung der genannten Laugen verwendet werden. Auf diese Weise kann auch in diesem Fall insbesondere eine weitere Nutzung entsprechender Ablauge vorgenommen werden, bevor diese einer Ablaugeeinheit zugeführt wird. Hierdurch verbessert sich, wie erwähnt, die Ausnutzung der Lauge. Diese Variante ist insbesondere in Figur 5 gezeigt.

Grundsätzlich ist es aber auch möglich, dass der vierte Waschmittelstrom aus Frischwasser, also beispielsweise demineralisiertem Wasser, oder aber auch beispielsweise aus dem aufgereinigten Wasser aus einer weiteren Prozesseinheit, insbesondere einer Essigsäurerückgewinnungseinheit, wie bereits oben zu der zweiten und dritten Variante erläutert, gebildet wird oder zumindest solches Wasser umfasst. Es ist aber auch möglich, den vierten Waschmittelstrom unter Verwendung von Flüssigkeit, die aus einem unteren Bereich des vierten Säulenteils entnommen wird, zu bilden. Sämtliche Alternativen können auch in Kombination eingesetzt werden.
Eine Anlage zur Herstellung von Ethylen, die dafür eingerichtet ist, einen Ethan und Sauerstoff enthaltenden Reaktionseinsatz zu bilden und einen Teil des Ethans und des Sauerstoffs in dem Reaktionseinsatz unter Erhalt eines Prozessgases durch oxidative Dehydrierung zu dem Ethylen und zu Essigsäure umzusetzen, wobei das Prozessgas den nicht umgesetzten Teil des Ethans und des Sauerstoffs, das Ethylen und die Essigsäure sowie Wasser enthält, und wobei Mittel vorgesehen sind, die dafür eingerichtet sind, das Prozessgas einer Wasserwäsche zu unterwerfen, ist ebenfalls Gegenstand der vorliegenden Erfindung.

Erfindungsgemäß ist in einer derartigen Anlage vorgesehen, dass zur Wasserwäsche eine Waschkolonne mit wenigstens zwei unterschiedlichen Kolonnenteilen bereitgestellt ist, Mittel vorgesehen sind, die dafür eingerichtet sind, das Prozessgas in die Waschkolonne einzuspeisen, und Mittel vorgesehen sind, die dafür eingerichtet sind, in die wenigstens zwei unterschiedlichen Kolonnenteile jeweils wässrige, flüssige Waschmittelströme einzuspeisen und dem Prozessgas entgegenzuschicken. Zu Merkmalen und Vorteilen einer entsprechenden Anlage sei auf die obigen Erläuterungen zu den Merkmalen und Vorteilen des Verfahrens verwiesen. Insbesondere ist eine derartige Anlage zur Durchführung eines Verfahrens gemäß den oben erläuterten spezifischen Ausgestaltungen eingerichtet und weist hierzu geeignete Mittel auf. Auch diesbezüglich sei auf die obigen Ausführungen verwiesen.

Die Erfindung wird nachfolgend unter Bezugnahme auf die beigefügten Zeichnungen näher erläutert, die unter anderem bevorzugte Ausführungsformen der vorliegenden Erfindung veranschaulichen.

### Kurze Beschreibung der Zeichnungen

Figur 1 veranschaulicht eine Anlage zur Herstellung von Olefinen gemäß einer Ausführungsform der Erfindung.
Figur 2 veranschaulicht eine Wasserwäsche gemäß einer nicht erfindungsgemäßen Ausführungsform in schematischer Darstellung.
Figuren 3A und 3B veranschaulichen Wasserwäschen gemäß Ausführungsformen der vorliegenden Erfindung in schematischer Darstellung.
Figuren 4A und 4B veranschaulichen Wasserwäschen gemäß Ausführungsformen der vorliegenden Erfindung in schematischer Darstellung.
Figur 5 veranschaulicht eine Wasserwäsche gemäß einer Ausführungsform der vorliegenden Erfindung in schematischer Darstellung.

### Ausführliche Beschreibung der Zeichnungen

In den nachfolgenden Figuren sind einander funktionell oder baulich entsprechende Elemente mit identischen Bezugszeichen angegeben und werden der Übersichtlichkeit halber nicht wiederholt erläutert. Werden nachfolgend Anlagenteile beschrieben, gelten die Erläuterungen zu diesen sinngemäß auch für die mittels dieser Anlagenteile implementierten Verfahrensschritte und umgekehrt.

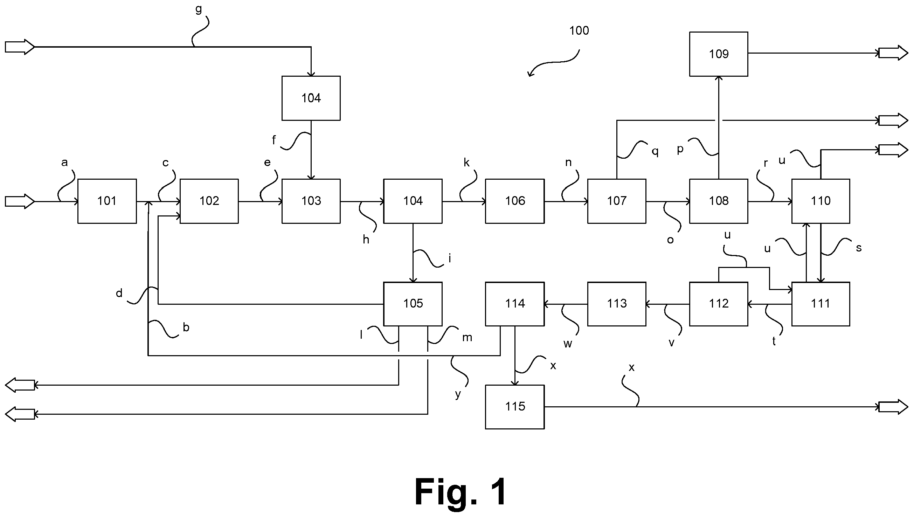

In Figur 1 ist eine Anlage zur Herstellung von Olefinen gemäß einer Ausführungsform der Erfindung in Form eines stark vereinfachten Anlagendiagramms veranschaulicht und insgesamt mit 100 bezeichnet. Wenngleich nachfolgend eine Anlage 100 zur ODH von Ethan (ODH-E) beschrieben wird, eignet sich, wie erwähnt, die vorliegende Erfindung auch zum Einsatz bei der ODH höherer Kohlenwasserstoffe. In diesem Fall gelten die nachfolgenden Erläuterungen entsprechend.

In der Anlage 100 wird einer Rektifikationseinheit 101 mit beispielsweise einer oder mehreren Rektifikationskolonnen ein Trenneinsatz in Form eines Stoffstroms a zugeführt und einer Rektifikation unterworfen. Der Trenneinsatz enthält im dargestellten Beispiel zumindest Ethan und höhere Kohlenwasserstoffe, insbesondere entsprechende höhere Paraffine. Der Rektifikationseinheit 101 können auch ein oder mehrere weitere Trenneinsätze zugeführt werden.

In der Rektifikationseinheit 101 wird der Trenneinsatz alleine oder zusammen mit dem oder den weiteren Trenneinsätzen unter Erhalt eines Trennprodukts, das Ethan enthält, aber arm an höheren Kohlenwasserstoffen ist, einer Rektifikation unterworfen. Das Trennprodukt wird in Form eines Stoffstroms c abgezogen und einer Vorwärmeinheit 102 zugeführt. In der Vorwärmeinheit 102 wird das Trennprodukt vorgewärmt, wobei der Vorwärmeinheit 102 im dargestellten Beispiel auch ein Wasser- oder Dampfstrom d zugeführt wird. Auch weitere Stoffströme können zugeführt werden. Zu dem Stoffstrom c kann ein weiterer, unten erläuterter Stoffstrom b zugespeist werden.

Ein aus der Vorwärmeinheit 102 abströmender Stoffstrom e wird zur Bildung eines Reaktionseinsatzes einer Reaktionseinheit 103 zugeführt. Der Reaktionseinsatz enthält aufgrund seiner Bildung unter Verwendung des Trennprodukts aus der Rektifikationseinheit 101 Ethan, ist aber arm an höheren Kohlenwasserstoffen. Der Reaktionseinsatz kann ferner ein oder mehrere Verdünnungsmittel wie Wasser oder Inertgase und weitere Komponenten enthalten. Diese können der Reaktionseinheit 103 auch in Form weiterer, nicht dargestellter Stoffströme zugeführt werden.

Der Reaktionseinheit 103 wird im dargestellten Beispiel ein sauerstoffhaltiger Stoffstrom f zugeführt. Dieser kann unter Verwendung einer Luftzerlegungsanlage 104 bereitgestellt werden. Der Luftzerlegungsanlage 104 wird hierzu ein Luftstrom g zugeführt. Bei dem sauerstoffhaltigen Stoffstrom f kann es sich um im Wesentlichen reinen Sauerstoff handeln, je nach Betrieb der Luftzerlegungsanlage 104 können jedoch auch Anteile an Stickstoff und an Edelgasen enthalten sein. Auf diese Weise ist es ebenfalls möglich, Verdünnungsmittel zuzuführen.

Aus der Reaktionseinheit 103 strömt ein Prozessgas in Form eines Prozessgasstroms h ab, in dem Ethylen enthalten ist, das in der Reaktionseinheit 103 durch ODH eines Teils des Ethans in dem Reaktionseinsatz gebildet wurden. Ferner enthält das Produktgemisch Essigsäure, die ebenfalls bei der ODH in der Reaktionseinheit 103 aus Ethan gebildet wurde, Wasser, Kohlenmonoxid, Kohlendioxid, nicht umgesetzten Sauerstoff, sowie das oder die Verdünnungsmittel und weitere Verbindungen, falls zugegeben oder zuvor in der Reaktionseinheit 103 gebildet.

Es versteht sich, dass Reaktionseinheit 103 einen, aber auch mehrere, beispielsweise parallel betriebene, Reaktoren aufweisen kann. In letzterem Fall werden diesen Reaktoren jeweils entsprechende Reaktionseinsätze, die gleich oder unterschiedlich zusammengesetzt sein können, sowie entsprechende sauerstoffhaltige Stoffströme f zugeführt werden, und es werden jeweils entsprechende Prozessgasströme h gebildet. Letztere können beispielsweise vereinigt und gemeinsam als Prozessgas den nachfolgend erläuterten Einheiten zugeführt werden.

Das Prozessgas wird in eine Quencheinheit 104 überführt, in der es, beispielsweise in einer Waschsäule, mit Waschwasser oder einer geeigneten wässrigen Lösung in Kontakt gebracht werden kann. In der Quencheinheit 104 wird das Prozessgas insbesondere abgekühlt und die in der Reaktionseinheit 103 gebildete Essigsäure wird aus dem Prozessgas ausgewaschen. Mit Essigsäure beladenes Prozesswasser strömt in Form eines Stoffstroms i aus der Quencheinheit 104 ab, das zumindest weitgehend von Essigsäure befreite Prozessgas in Form eines Stoffstroms k.

In einer optionalen Essigsäurerückgewinnungseinheit 105 wird aus dem mit Essigsäure beladenen Prozesswasser Essigsäure als Eisessig abgetrennt, welcher als Stoffstrom I aus der Anlage 100 ausgeführt wird. In der Essigsäurerückgewinnungseinheit 105 ebenfalls rückgewonnenes reines Prozesswasser kann in Form des bereits erläuterten Stoffstroms d der Vorwärmeinheit 102 zugeführt werden. Das dem Reaktor zugeführte Prozesswasser kann auch teilweise oder vollständig in Form von extern zugeführtem Frischwasser bereitgestellt werden. Nicht mehr brauchbares oder benötigtes Wasser kann in Form eines Abwasserstroms m aus der Anlage 100 ausgeführt und einer Abwasseraufbereitung zugeführt werden.

Das in Form des Stoffstroms k vorliegende, zumindest weitgehend von Essigsäure befreite Prozessgas wird in einer Verdichtungseinheit 106 auf ein geeignetes Druckniveau, beispielsweise 15 bis 25 bar, verdichtet und in Form eines verdichteten Stoffstroms n einer Aminwäscheeinheit 107 zugeführt. In dieser werden insbesondere Teile des in dem Prozessgas enthaltenen Kohlendioxids ausgewaschen. Nach Regenerierung des Amins kann das ausgewaschene Kohlendioxid in Form eines Stoffstroms q aus der Anlage ausgeführt werden.

Das derart teilweise von Kohlendioxid befreite Prozessgas wird in Form eines Stoffstroms o in eine Laugenwäscheeinheit 108 überführt und dort weiter von Kohlendioxid gereinigt. In der Laugenwäscheeinheit 108 fällt Ablauge an, die in Form eines Stoffstroms p in eine Ablaugebehandlungseinheit 109 überführt und schließlich aus der Anlage ausgeführt wird.

Das in der Laugenwäscheeinheit 108 weiter gereinigte Prozessgas wird in Form eines Stoffstroms r in eine Vorkühl- und Trockeneinheit 110 überführt, wo es insbesondere von Restwasser befreit werden kann. Das getrocknete Prozessgas wird in Form eines Stoffstroms s in eine Tieftemperatureinheit 111 überführt und anschließend in weiter abgekühlter Form in Form eines oder mehrerer Stoffströme t in eine Demethanisierungseinheit 112. In der Tieftemperatureinheit 111 und der Demethanisierungseinheit 112 werden tiefer als Ethylen siedende Komponenten, darunter insbesondere Kohlenmonoxid und Sauerstoff, aus dem Prozessgas abgetrennt, wobei der Rest in kondensierter Form verbleibt. Sind in dem Prozessgas höhere Kohlenwasserstoffe enthalten, die bei der ODH in der Reaktionseinheit 103 als Nebenprodukt gebildet wurden, gehen diese ebenfalls in das Kondensat über.

Die abgetrennten, tiefer als Ethylen siedenden Komponenten werden in Form eines oder mehrerer Stoffströme u durch die Tieftemperatureinheit 111 und die Vorkühl- und Trockeneinheit 110 zurückgeführt, dort ggf. mit weiteren entsprechender Stoffströme vereinigt, zu Kühlzwecken genutzt, und aus der Anlage 100 ausgeführt. Bei Bedarf werden die Kohlenwasserstoffe mit zwei und ggf. mehr Kohlenstoffatomen in Form eines Stoffstroms v einer Hydriereinheit 113 zugeführt, in der insbesondere Acetylen, das ebenfalls bei der ODH in der in der Reaktionseinheit 103 als Nebenprodukt gebildet wurde, hydriert werden kann. Nach der Hydrierung wird der nun mit w bezeichnete Stoffstrom in eine Ethylenabtrenneinheit 114 überführt.

In der Ethylenabtrenneinheit 114 wird Ethylen zumindest weitgehend von anderen Komponenten abgetrennt und kann in Form eines Stoffstroms x nach Nutzung in einer Ethylenkälteeinheit 115 gasförmig aus der Anlage 100 ausgeführt werden. Die übrigen Komponenten, überwiegend Ethan und ggf. höhere Kohlenwasserstoffe, werden in Form eines Stoffstroms y abgezogen und in Form des Stoffstroms b in die Vorwärmeinheit 101 zurückgeführt.

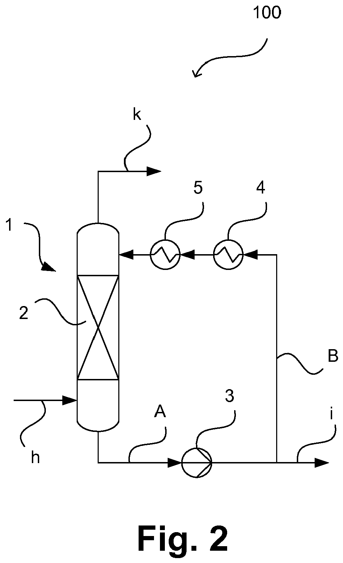

Figur 2 veranschaulicht eine Wasserwäsche gemäß einer nicht erfindungsgemäßen Ausführungsform, die nachfolgenden Figuren 3A und 3B, 4A und 4B sowie 5 veranschaulichen jeweils Wasserwäschen gemäß Ausführungsformen der vorliegenden Erfindung in schematischer Darstellung. Die in den genannten Figuren gezeigten Elemente 1 bis 9 erfüllen jeweils gleiche oder vergleichbare Funktionen und sind daher grundsätzlich vergleichbar aufgebaut. Sie können jedoch jeweils in unterschiedlichen Ausgestaltungen, beispielsweise in unterschiedlichen Bemaßungen und einer unterschiedlichen Anzahl, vorgesehen sein.

Die mit den gleichen Großbuchstaben bezeichneten Stoffströme sind ebenfalls grundsätzlich vergleichbar. Die in den genannten gezeigten Wasserwäschen können als die Quencheinheit 104 gemäß Figur 1 oder als Teil einer entsprechenden Quencheinheit verwendet werden, ihre Einbindung ergibt sich über die hier wie dort mit h, i und k bezeichneten Stoffströme.

Wie mehrfach erwähnt, betrifft die vorliegende Erfindung ein Verfahren zur Herstellung von Ethylen, bei dem ein Ethan und Sauerstoff enthaltender Reaktionseinsatz gebildet und ein Teil des Ethans und des Sauerstoffs in dem Reaktionseinsatz unter Erhalt eines Prozessgases durch oxidative Dehydrierung zu dem Ethylen und zu Essigsäure umgesetzt wird, wobei das Prozessgas den nicht umgesetzten Teil des Ethans und des Sauerstoffs, das Ethylen und die Essigsäure sowie Wasser enthält.

Dieses Prozessgas, das insbesondere auch noch weitere Komponenten wie beispielsweise Kohlenmonoxid und Kohlendioxid enthalten kann, wird in der in Figur 2 veranschaulichten nicht erfindungsgemäßen Variante auf einem Temperaturniveau von beispielsweise ca. 140 °C in Form des wie in Figur 1 mit h bezeichneten Stoffstroms einer Waschsäule 1 zugeführt.

Die Waschsäule 1 umfasst einen Austauschbereich 2, der geeignete Böden und/oder Schüttungen aufweisen kann. Aus dem Sumpfbereich der Waschsäule 1 wird eine flüssige Fraktion auf einem Temperaturniveau von beispielsweise ca. 80 bis 100 °C in Form eines Stoffstroms A entnommen und teilweise mittels einer Pumpe 3 in Form eines Stoffstroms B durch einen Wärmetauscher 4 und einen Wärmetauscher 5 und zurück in einen Kopfbereich der Waschsäule 1 geführt.

Auf diese Weise wird ein wässriger, flüssiger Waschmittelstrom gebildet und dem Prozessgas entgegengeschickt. Die im Sumpf der Waschsäule 1 anfallende flüssige Fraktion stellt im Wesentlichen ein Wasser-Essigsäure-Gemisch dar. In Form des wie in Figur 1 mit i bezeichneten Stoffstroms kann eine Ausschleusung des überschüssigen Wasser-Essigsäure-Gemischs erfolgen. In Form des wie in Figur 1 mit k bezeichneten Stoffstroms kann ein gekühltes Prozessgas abgezogen werden.

Über den Wärmetauscher 4 wird eine Wärmeintegration vorgenommen und die flüssige Fraktion auf ein Temperaturniveau von beispielsweise 50 bis 90 °C gekühlt. In dem Wärmetauscher 5 wird die flüssige Fraktion mittels Kühlwasser auf ein Temperaturniveau von beispielsweise 20 bis 50 °C ge kühlt.

Gemäß der in Figur 2 veranschaulichten Variante wird ein Quench, also eine rasche Abkühlung, mit den Zielen einer Wärmeintegration und einer größtmöglichen Abkühlung bei geringem Druckverlust vorgenommen. Es wird jedoch typischerweise eine vergleichsweise geringe Abreicherung an Essigsäure erzielt.

Dies wird jedoch in den in den nachfolgenden Figuren 3A und 3B, 4A und 4B sowie 5 veranschaulichten Ausführungsformen der Erfindung erzielt, in denen die Wasserwäsche umfasst, das Prozessgas jeweils in eine Waschkolonne einzuspeisen, in die in wenigstens zwei unterschiedlichen Kolonnenteilen jeweils wässrige, flüssige Waschmittelströme eingespeist und dem Prozessgas entgegengeschickt werden.

In der in Figur 3A veranschaulichten Ausführungsform ist die Waschkolonne mit 10 bezeichnet. Diese umfasst zwei Austauschbereiche 2, die jeweils geeignete Böden und/oder Schüttungen aufweisen können. Auf diese Weise wird die Waschkolonne 10 geteilt. Ein Teilstrom C der mittels der Pumpe 3 geförderten Gesamtumlaufmenge des Stoffstroms B wird durch den Wärmetauscher 4 zwischen den beiden Austauschbereichen 2 in die Waschkolonne 10 eingespeist. Ein weiterer Teilstrom C' der mittels der Pumpe 3 geförderten Gesamtumlaufmenge wird durch den Wärmetauscher 5 und einen weiteren, insbesondere wärmeintegrierten, Wärmetauscher 5a in den Kopfbereich der Waschkolonne 10 eingespeist.

Die Temperaturniveaus entsprechen dabei im Wesentlichen jenen, die zuvor bezüglich der beiden Waschmittelströme erläutert wurden. In dem Wärmetauscher 5 kann dabei gemäß der in Figur 3A veranschaulichten Ausführungsform auch eine Luftkühlung vorgenommen werden.

Durch die in Figur 3A veranschaulichte Ausführungsform kann gegenüber der in Figur 2 veranschaulichten Ausführungsform das Temperaturprofil der Kolonne besser eingestellt und somit auch ein höherer Grad der Wärmeintegration erreicht werden, da mehr Energie auf höherem Temperaturniveau abgeführt werden kann.

In der in Figur 3B veranschaulichten Ausführungsform sind zwei komplett voneinander getrennte Kreisläufe vorgesehen, die es insbesondere ermöglichen, ein Gefälle in der Essigsäurekonzentration von unten nach oben zu erreichen und somit den Essigsäureeintrag in eine nachfolgende Verdichtungseinheit wie die Verdichtungseinheit 106 entsprechend Figur 1 zu reduzieren. Zusätzlich wird durch diese Maßnahme das Potential zur Wärmeintegration noch weiter gesteigert.

Hierzu ist die hier mit 20 bezeichnete Waschkolonne mittels eines Flüssigkeitssperrbodens 6, insbesondere mittels eines Kaminhalsbodens, unterteilt. Die aus dem Sumpf der Waschkolonne 20 in Form eines Stoffstroms D abgezogene Flüssigkeit wird teilweise über den Wärmetauscher 4 in Form eines Stoffstroms E unterhalb des Flüssigkeitssperrbodens 21 in einen unteren Kolonnenteil der Waschkolonne 20 eingespeist.

Eine sich auf dem Flüssigkeitssperrboden 6 sammelnde Flüssigkeit wird auf einem Temperaturniveau von beispielsweise 40 bis 70 °C in Form eines Stoffstroms F abgezogen und in Form eines Stoffstroms G mittels einer Pumpe zum Teil durch den Wärmetauscher 7 geführt und am Kopf der Waschkolonne 20 eingespeist. Ein weiterer Anteile des Stoffstroms F wird in Form eines Stoffstroms G' in den unteren Waschmittelkreislauf geleitet und/oder ausgeführt. Die Ausfuhr kann, wie nicht gesondert gezeigt, getrennt zu der vom unteren Kreislauf erfolgen, bei der ein Anteil des Stoffstroms D als Stoffstrom i ausgeführt wird, oder die beiden Ausführströme werden gemischt als Stoffstrom i aus der Anlage geführt.

In den in den Figuren 4A und 4B gezeigten Ausführungsformen wird gegenüber den in den Figuren 2, 3A und 3B gezeigten Ausführungsformen ein zusätzlicher Kolonnenteil hinzugefügt, d.h. die jeweils mit 30 bzw. 40 bezeichneten Waschkolonnen sind in ihrem hier mit 31 bzw. 42 bezeichneten unteren Teil wie die in den Figuren 2, 3A und 3B dargestellten Waschkolonnen ausgebildet und umfassen entsprechende Waschmittelkreisläufe, Einbauten usw.

Dies ist lediglich aus Gründen der Übersichtlichkeit nicht in allen Kombinationen gezeigt. So umfasst die in Figur 4A gezeigte Ausführungsform im dargestellten Beispiel den unteren Teil 31 gemäß der in Figur 3A gezeigten Ausführungsform, der untere Teil 31 kann jedoch auch gemäß der in Figur 3B gezeigten Ausführungsform ausgebildet sein. Entsprechend umfasst die in Figur 4B gezeigte Ausführungsform im dargestellten Beispiel den unteren Teil 41 gemäß der in Figur 3B gezeigten Ausführungsform, der untere Teil 41 kann jedoch auch gemäß der in Figur 3A gezeigten Ausführungsform ausgebildet sein.

Die jeweils hinzugefügten Kolonnenteile sind mit 32 bzw. 42 bezeichnet. Sie sind mittels eines weiteren Flüssigkeitssperrbodens von den unteren Teilen 31 bzw. 41 abgegrenzt. Oberhalb dieses Flüssigkeitssperrbodens 6 wird jeweils Flüssigkeit in Form eines Stoffstroms H abgezogen.

Gemäß der in Figur 4A veranschaulichten Ausführungsform ist ein gepumpter Kreislauf mit einer Pumpe 8 vorgesehen, mittels derer zumindest ein Teil des Stoffstroms H in Form eines Stoffstroms I auf einem Temperaturniveau von beispielsweise 20 bis 50 °C auf den Kopfbereich des Kolonnenteils 32 zurückgeführt werden kann, gemischt mit einem Stoffstrom L von frischem, demineralisierten Wasser oder aber auch beispielsweise aufgereinigtem Wasser aus einer Essigsäurerückgewinnungseinheit, beispielsweise der Essigsäurerückgewinnungseinheit 105 entsprechend Figur 1 mit beispielsweise ca. 0 bis 5.000 Gew.-ppm Essigsäure. Überschüssige Flüssigkeit wird, beispielsweise füllstandsgeregelt, in Form eines Stoffstroms K dem Stoffstrom C zugespeist oder anderweitig abgeleitet.

Gemäß der in Figur 4B veranschaulichten Ausführungsform wird ausschließlich frisches, demineralisiertes Wasser oder aber auch beispielsweise aufgereinigtes Wasser aus einer Essigsäurerückgewinnungseinheit, beispielsweise der Essigsäurerückgewinnungseinheit 105 entsprechend Figur 1 mit beispielsweise ca. 0 bis 5.000 Gew.-ppm Essigsäure und auf einem Temperaturniveau von beispielsweise 20 bis 50 °C in Form eines Stoffstroms L am Kopf de s Kolonnenteils 42 aufgegeben. Überschüssige Flüssigkeit wird, beispielsweise füllstandsgeregelt, in Form eines Stoffstroms K dem Stoffstrom C zugespeist oder anderweitig abgeleitet. Der Stoffstrom H wird beispielsweise füllstandsgeregelt zumindest zum Teil in Form eines Stoffstroms M dem Stoffstrom G zugespeist oder anderweitig abgeleitet.

In Figur 5 ist eine Ausführungsform veranschaulicht, bei der die in den Figuren 4A und 4B gezeigten Ausführungsformen nochmals erweitert sind. Die Teile 31 bzw. Sektionen 41 und 32 bzw. 42, die in den Figuren 4A und 4B veranschaulicht sind, können hierbei in beliebiger Kombination vorgesehen sein und sind aus Gründen der Allgemeingültigkeit in der Figur 5 nicht detailliert gezeigt.

Wiederum ist der oberste Kolonnenteil der hier mit 50 bezeichneten Waschkolonne mit einem Flüssigkeitssperrboden 6 von den darunter liegenden Kolonnenteilen 31, 32, 41 und 42 abgetrennt. Hierdurch wird ein weiterer Kolonnenteil hinzugefügt, der in einen über eine Pumpe 9 gepumptem Kreislauf integriert ist, in den entweder ein Ablaugestrom aus einer nachgeschalteten Prozesseinheit wie beispielsweise der Laugenstrom p aus der Laugenwäscheeinheit 108 entsprechend Figur 1, eine frische Laugenlösung oder eine Kombination aus beiden gespeist wird. Ein entsprechender Stoffstrom ist in Figur 5 mit N bezeichnet.

Zusammen mit einem Kreislaufstrom O wird der Stoffstrom M als Stoffstrom P am Kopf der Waschkolonne 50 aufgegeben. Oberhalb des Flüssigkeitssperrbodens 6 wird beladene Lauge Q abgezogen. Diese kann teilweise in Form des Kreislaufstroms O verwendet und zum Teil als Stoffstrom R in eine Ablaugebehandlungseinheit wie die Ablaugebehandlungseinheit 109 gemäß Figur 1 ausgeleitet werden.

Auf diese Weise wird zusätzlich zur Rückwaschung mit Wasser eine chemisch basierte Wäsche vorgesehen, die die über Kopf gehende Essigsäuremenge noch weiter reduziert und damit nachgeschaltete Einheiten schont.

## Patentansprüche

1. Verfahren zur Herstellung von Ethylen, bei dem ein Ethan und Sauerstoff enthaltender Reaktionseinsatz gebildet und ein Teil des Ethans und des Sauerstoffs in dem Reaktionseinsatz unter Erhalt eines Prozessgases durch oxidative Dehydrierung zu Ethylen und zu Essigsäure umgesetzt wird, wobei das Prozessgas den nicht umgesetzten Teil des Ethans und des Sauerstoffs, das Ethylen und die Essigsäure sowie Wasser enthält, und wobei das Prozessgas einer Wasserwäsche unterworfen wird, **dadurch gekennzeichnet, dass** die Wasserwäsche umfasst, das Prozessgas in eine Waschkolonne (10, 20, 30, 40, 50) einzuspeisen, in die in wenigstens zwei unterschiedlichen Kolonnenteilen jeweils wässrige, flüssige Waschmittelströme eingespeist und dem Prozessgas entgegengeschickt werden.

2. Verfahren nach Anspruch 1, bei dem eine Waschkolonne (10, 20, 30, 40, 50) mit einem ersten Kolonnenteil und einem zweiten Kolonnenteil verwendet wird, wobei der zweite Kolonnenteil oberhalb des ersten Kolonnenteils angeordnet ist, das Prozessgas in einen unteren Bereich des ersten Kolonnenteils eingespeist und teilweise oder vollständig durch den ersten Kolonnenteil, von dem ersten Kolonnenteil in den zweiten Kolonnenteil und durch den zweiten Kolonnenteil aufsteigen gelassen wird, und in einem oberen Bereich des ersten Kolonnenteils ein erster flüssiger Waschmittelstrom und in einem oberen Bereich des zweiten Kolonnenteils ein zweiter flüssiger Waschmittelstrom eingespeist wird.

3. Verfahren nach Anspruch 2, bei dem der erste Waschmittelstrom auf einem ersten Temperaturniveau in den ersten Kolonnenteil und der zweite Waschmittelstrom auf einem zweiten Temperaturniveau in den zweiten Kolonnenteil eingespeist wird.

4. Verfahren nach Anspruch 3, bei dem das erste Temperaturniveau bei 50 bis 90 °C und/oder das zweite Temperaturniveau bei 20 bis 50 °C liegt.

5. Verfahren nach einem der Ansprüche 2 bis 4, bei dem aus einem unteren Bereich des ersten Kolonnenteils Flüssigkeit entnommen und teilweise oder vollständig zur Bildung des ersten und/oder des zweiten Waschmittelstroms verwendet wird.

6. Verfahren nach einem der Ansprüche 2 bis 4, bei dem der erste Kolonnenteil durch einen Flüssigkeitssperrboden 6 von dem zweiten Kolonnenteil getrennt ist.

7. Verfahren nach Anspruch 6, bei dem aus einem unteren Bereich des ersten Kolonnenteil Flüssigkeit entnommen und teilweise oder vollständig bei der Bildung des ersten Waschmittelstroms verwendet wird, und bei dem aus einem unteren Bereich des zweiten Kolonnenteils Flüssigkeit entnommen wird.

8. Verfahren nach Anspruch 7, bei dem der erste und der zweite Waschmittelstrom überwiegend Wasser enthalten, und bei dem der erste Waschmittelstrom einen höheren Essigsäuregehalt aufweist als der zweite Waschmittelstrom.

9. Verfahren nach einem der Ansprüche 2 bis 8, bei dem eine Waschkolonne (30, 40, 50) mit einem dritten Kolonnenteil oberhalb des zweiten Kolonnenteils verwendet wird, wobei das Prozessgas teilweise oder vollständig von dem zweiten Kolonnenteil in den dritten Kolonnenteil und durch den dritten Kolonnenteil aufsteigen gelassen wird, in einem oberen Bereich des dritten Kolonnenteils ein dritter flüssiger Waschmittelstrom eingespeist wird.

10. Verfahren nach Anspruch 9, bei dem aus dem unteren Bereich des dritten Kolonnenteils Flüssigkeit entnommen wird und teilweise oder vollständig bei der Bildung des zweiten Waschmittelstroms verwendet wird.

11. Verfahren nach Anspruch 9, bei dem aus dem unteren Bereich des dritten Kolonnenteils Flüssigkeit entnommen wird und teilweise oder vollständig bei der Bildung des dritten Waschmittelstroms verwendet wird.

12. Verfahren nach einem der Ansprüche 9 bis 11, bei dem bei der Bildung des dritten Waschmittelstroms zumindest teilweise ein extern zugeführter Strom verwendet wird, der überwiegend aus Wasser besteht und höchstens 5.000 Gew.-ppm Essigsäure enthält.

13. Verfahren nach einem der Ansprüche 2 bis 12, bei dem eine Waschkolonne (50) mit einem vierten Kolonnenteil oberhalb des dritten Kolonnenteils verwendet wird, wobei das Prozessgas teilweise oder vollständig von dem dritten Kolonnenteil in den vierten Kolonnenteil und durch den vierten Kolonnenteil aufsteigen gelassen wird, in einem oberen Bereich des vierten Kolonnenteils ein vierter flüssiger Waschmittelstrom eingespeist wird, und aus einem unteren Bereich des vierten Kolonnenteils Flüssigkeit entnommen wird.

14. Verfahren nach Anspruch 11, bei dem ein wässriger Laugen- oder Ablaugenstrom als der vierte Waschmittelstrom verwendet wird.

15. Anlage (100) zur Herstellung von Ethylen, die dafür eingerichtet ist, einen Ethan und Sauerstoff enthaltenden Reaktionseinsatz zu bilden und ein Teil des Ethans und des Sauerstoffs in dem Reaktionseinsatz unter Erhalt eines Prozessgases durch oxidative Dehydrierung zu dem Ethylen und zu Essigsäure umzusetzen, wobei das Prozessgas den nicht umgesetzten Teil des Ethans und des Sauerstoffs, das Ethylen und die Essigsäure sowie Wasser enthält, und wobei Mittel vorgesehen sind, die dafür eingerichtet sind, das Prozessgas einer Wasserwäsche zu unterwerfen, **dadurch gekennzeichnet, dass** zur Wasserwäsche eine Waschkolonne (10, 20, 30, 40, 50) mit wenigstens zwei unterschiedlichen Kolonnenteilen bereitgestellt ist, Mittel vorgesehen sind, die dafür eingerichtet sind, das Prozessgas in die Waschkolonne (10, 20, 30, 40, 50) einzuspeisen, und Mittel vorgesehen sind, die dafür eingerichtet sind, in die wenigstens zwei unterschiedlichen Kolonnenteile jeweils wässrige, flüssige Waschmittelströme einzuspeisen und dem Prozessgas entgegenzuschicken.
